# EUROPEAN PATENT APPLICATION

(11) **EP 4 470 567 A1**
(43) Date of publication of application: **04.12.2024**
(21) Application number: 23746036.5
(22) Date of filing: 16.01.2023
(51) Int. Cl.: A61K 47/68, C07D 491/22, A61K 9/08, A61K 9/19, A61P 35/00, A61K 31/506

(54) **PHARMACEUTICAL COMPOSITION, METHOD FOR PREPARING SAME, AND USE THEREOF**

(30) Priority: 28.01.2022 CN 202210106842
(71) Applicant: Sichuan Kelun-Biotech Biopharmaceutical Co., Ltd., Chengdu, Sichuan 611138 (CN)
(72) Inventor: SHAN, Wei, Chengdu, Sichuan 611138 (CN); LOU, Ruyun, Chengdu, Sichuan 611138 (CN); HUANG, Haiyu, Chengdu, Sichuan 611138 (CN); SONG, Saina, Chengdu, Sichuan 611138 (CN); LEI, Fubin, Chengdu, Sichuan 611138 (CN)
(74) Representative: Jones Day
(86) International application number: PCT/CN2023/072262
(87) International publication number: WO 2023/143154

(57) **Abstract**

Disclosed are a pharmaceutical composition, and a preparation method and use thereof. The composition includes the following components: an antibody-drug conjugate, a buffer system, a lyoprotectant, and a surfactant. The pharmaceutical composition is used for preparing anti-tumor drugs. The antibody-drug conjugate may be a conjugate A.

## Description

The present application is based on and claims the priority of the Chinese application No. 202210106842.X filed on January 28, 2022, the disclosure of which is incorporated into the present application herein in its entirety.

### TECHNICAL FIELD

The present application relates to the technical field of pharmaceutical formulations, and in particular to a pharmaceutical composition of an antibody-drug conjugate, and a preparation method and use thereof.

### BACKGROUND

Antibody-drug conjugates (ADCs) are products obtained by coupling antibodies and drugs, and are used for treating tumors or other diseases. An antibody moiety of a conjugate can specifically bind to the antigen on a target cell, while a drug moiety of the conjugate can exert cytotoxic or other therapeutic effects on the target cell.

A monoclonal antibody has a complex advanced structure and poor self-stability. In ADC drugs, the physical and chemical properties of antibodies change after being coupled to drugs, and their stability also decreases accordingly. Also, compared with antibodies, ADCs are heterogeneous and have more complex structures because they are coupled with small-molecule drug moieties. This is also one of the reasons why ADCs are less stable and more prone to aggregation, which greatly increases the risks in their clinical applications.

Patent No. CN201880069543.5 records an antibody-drug conjugate with good anti-tumor activity and safety. The conjugate is formed by opening 4 pairs of disulfide bonds between antibody chains and coupling them to toxin linkers via sulfhydryl groups. Since the interchain disulfide bonds are destroyed, the antibody stability is worse. Also, the small-molecule toxin drug moiety is hydrophobic, and up to 8 toxin small molecules can be coupled to a single antibody molecule. Therefore, the conjugate has strong overall hydrophobicity and worse stability. Furthermore, the conjugate has a cleavable connection site, and thus has poor stability in an aqueous solution. Taking the aforementioned several factors into consideration, it is necessary to develop a pharmaceutical composition of the conjugate to improve its stability.

### SUMMARY OF THE INVENTION

The present application provides a pharmaceutical composition of an antibody-drug conjugate, and a preparation method and use thereof. The pharmaceutical composition can significantly improve the stability of the antibody-drug conjugate without adding a preservative, thereby improving the compliance of clinical medication. Moreover, the present application adopts a large-volume freeze-drying process to prepare the pharmaceutical composition, thereby improving production capacity.

Specifically, the present application provides a pharmaceutical composition including the following components:
an antibody-drug conjugate, a buffer system, a lyoprotectant, and a surfactant.

In some embodiments, the buffer system is selected from one or more of histidine hydrochloride/histidine, succinic acid/sodium succinate, and citric acid/sodium citrate; and preferably histidine hydrochloride/histidine.

In some embodiments, a concentration range of the buffer system is 5-50 mM.

In some embodiments, the lyoprotectant is selected from one or more of sucrose, trehalose, sorbitol, and mannitol; and preferably sucrose.

In some embodiments, the surfactant is selected from polysorbate or poloxamer, preferably polysorbate 20 or polysorbate 80, and further preferably polysorbate 20.

In some embodiments, the conjugate has a structure of formula (I):

{D-[L₁-(L₂)m₁-(L₃)m₂-(L₄)m₃-E]}_{γ}-A formula (I)

wherein
L₁ is 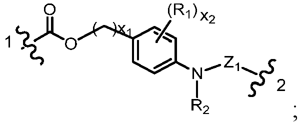 R₁ and R₂ are each independently hydrogen (e.g., protium or deuterium), halogen, carboxylic acid, sulfonic acid, cyano, C₁₋₆ alkyl, halogenated C₁₋₆ alkyl, cyano-substituted C₁₋₆ alkyl (e.g., -CH₂CN), C₁₋₆ alkoxy, C₂₋₁₀alkenyl or C₂₋₁₀ alkynyl; Z₁ is an amino acid or a peptide composed of 2-10 amino acids; x₁ and x₂ are each independently 0, 1, 2, 3, 4, 5 or 6; and the position 1 of L₁ is linked to D, and the position 2 of L₁ is linked to L₂;
L₂ is 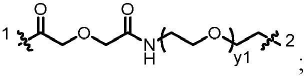 wherein y₁ is 0, 1, 2, 3, 4, 5, 6, 7, 8, 9 or 10; and the position 1 of L₂ is linked to the L₁, and the position 2 of L₂ is linked to L₃;
L₃ is a 5-12 membered heteroaromatic ring;
L4 is 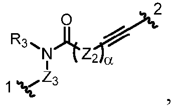 wherein Z₂ is selected from C₁₋₆ alkylene, C₂₋₁₀ alkenylene, C₂₋₁₀ alkynylene, and C₃₋₈ cycloalkylene; R₃ is selected from H and C₁₋₆ alkyl; Z₃ is absent or C₁₋₆ alkylene; or R₃ and Z₃, together with a nitrogen atom to which they are attached, form a 4-8 membered heterocyclic group; α is 0, 1, 2, 3, 4, 5 or 6, and the position 2 of L₄ is linked to E, and the position 1 of L₄ is linked to L₃;
E is 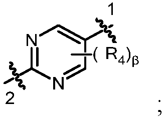 wherein each R₄ is independently hydrogen (e.g., protium or deuterium), β is 0, 1 or 2, and the position 2 of E is linked to A, and the position 1 of E is linked to L₄;
mi, m₂ and m₃ are each independently 0, 1, 2, 3, 4, 5, 6, 7, 8, 9 or 10;
D is a bioactive molecular fragment;
γ refers to the number of {D-[L₁-(L₂)ₘ₁-(L₃)ₘ₂-(L₄)ₘ₃-E]} moieties that form a thioether bond with the sulfhydryl group of A, which is selected from 1-10; preferably, γ is selected from 3-8 (e.g. 3, 4, 5, 6, 7 or 8);
A is an anti-Trop-2 monoclonal antibody or an antigen-binding fragment thereof.

In some embodiments, the antibody-drug conjugate has a structure as shown in the conjugate A below: 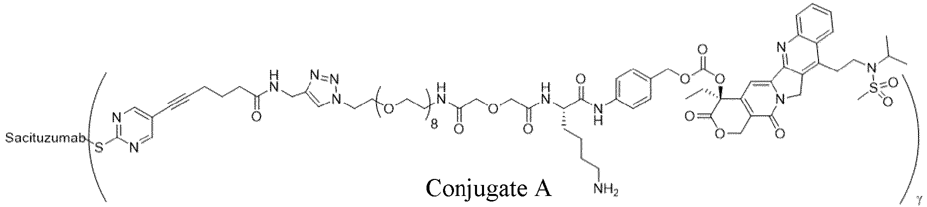 wherein γ is selected from 1-10.

In some embodiments, in the conjugate A, γ is selected from 3-8 (e.g. 3, 4, 5, 6, 7 or 8).

In some embodiments, a DAR value of the conjugate is 1-10; further preferably, the DAR value is 3-8; and for example, the DAR value is 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0.

In some embodiments, the pharmaceutical composition includes the following components:
an antibody-drug conjugate, histidine hydrochloride, histidine, sucrose, and polysorbate 20; and preferably, the antibody-drug conjugate is a conjugate A.

In some embodiments, the pharmaceutical composition has a pH in a range of 4.5-7.0, preferably 5.0-6.5, more preferably 5.4-6.3, more preferably 5.7-6.3, and most preferably 6.0 ± 0.3.

In some embodiments, the pharmaceutical composition contains:
5-40 mg/ml, preferably 15-25 mg/ml, and further preferably 18-22 mg/ml of the antibody-drug conjugate;
0.1-3.5 mg/ml, preferably 0.4-1.2 mg/ml, and further preferably 0.6-0.8 mg/ml of the histidine;
0.1-5.5 mg/ml, preferably 0.5-2.0 mg/ml, and further preferably 1.0-1.3 mg/ml of the histidine hydrochloride;
30-150 mg/ml, preferably 60-120 mg/ml, and further preferably 70-90 mg/ml of the lyoprotectant;
   and/or
0.01-0.50 mg/ml, preferably 0.05-0.30 mg/ml, and further preferably 0.15-0.25 mg/ml of the surfactant;
In some embodiments, the pharmaceutical composition is in unit dosage form.

In some embodiments, the unit dosage form contains 40-400 mg, e.g. 200 mg of the antibody-drug conjugate.

In some embodiments, the pharmaceutical composition has a specification of 40-400 mg of an active ingredient per vial, and preferably 200 mg of an active ingredient per vial.

In some embodiments, the pharmaceutical composition is a lyophilized preparation, e.g. lyophilized powder.

In some embodiments, the pharmaceutical composition contains 20 mg/ml of the conjugate A, 1.11 mg/ml of the histidine hydrochloride, 0.73 mg/ml of the histidine, 80 mg/ml of the sucrose, and 0.2 mg/ml of the polysorbate 20.

The present application further provides a method for preparing the pharmaceutical composition, including the following steps:
adding the antibody-drug conjugate, the buffer system, the lyoprotectant and the surfactant into water for injection for lyophilizing to obtain the pharmaceutical composition.

In some embodiments, the lyophilizing includes feeding, pre-freezing, vacuuming, subliming and desorption drying steps.

In some embodiments, the pre-freezing step is conducted at a temperature range of -50°C-0°C for a time period of 400-800 minutes.

In some embodiments, the subliming step is conducted at a temperature range of -30-0°C and a pressure of 0-100 pa for a time period of 2,000-5,000 minutes.

In some embodiments, the desorption drying step is conducted at a temperature range of 0 - - 50°C and a pressure of 0-100 pa for a time period of 200-2,000 minutes.

The present application further provides a solid preparation obtained by lyophilizing the pharmaceutical composition according to any one of embodiments herein.

In some embodiments, the lyophilizing includes the following steps:
adding the antibody-drug conjugate, the buffer system, the lyoprotectant and the surfactant into water for injection for lyophilizing to obtain the solid preparation.

In some embodiments, the lyophilizing includes feeding, pre-freezing, vacuuming, subliming and desorption drying steps.

In some embodiments, the pre-freezing is conducted at -50°C-0°C for a time period of 400-800 minutes.

In some embodiments, the subliming is conducted at -30-0°C and a pressure of 0-100 pa for a time period of 2,000-5,000 minutes.

In some embodiments, the desorption drying is conducted at 0 - -50°C and a pressure of 0-100 pa for a time period of 200-2,000 minutes.

The present application further provides a reconstituted liquid preparation, which is obtained by redissolving the pharmaceutical composition or solid preparation according to any one of embodiments herein in a solvent.

In some embodiments, the solvent is selected from water for injection, normal saline and 5% dextrose in water.

In some embodiments, the solvent is water for injection.

The present application further provides use of the pharmaceutical composition, solid preparation, or reconstituted liquid preparation according to any one of embodiments herein in preparation of a drug for treating tumors.

In some embodiments, the tumor disease is an unresectable locally advanced or metastatic solid tumor for which standard treatment fails, or there is no standard treatment scheme, or standard treatment is not applicable at this stage.

In some embodiments, the tumor is selected from breast cancer, gastric cancer, lung cancer, ovarian cancer, urothelial cancer, esophageal cancer, liver cancer, colorectal cancer, cervical cancer, endometrial cancer, pancreatic cancer, bladder cancer, or brain tumor; and preferably breast cancer (e.g. triple-negative breast cancer or Her2-positive breast cancer), ovarian cancer (e.g. ovarian epithelial cancer), gastric cancer, lung cancer, pancreatic cancer, bladder cancer, or urothelial cancer; and more preferably, the tumor disease is triple-negative breast cancer, Her2-positive breast cancer, ovarian cancer, gastric cancer, lung cancer or pancreatic cancer.

In some embodiments, the tumor is triple-negative breast cancer, Her2-positive breast cancer, ovarian cancer or gastric cancer.

The present application further provides a method for treating tumors, including the step of administering to a subject a therapeutically effective amount of the pharmaceutical composition, solid preparation, or reconstituted liquid preparation according to any one of embodiments herein.

In some embodiments, the tumor disease is an unresectable locally advanced or metastatic solid tumor for which standard treatment fails, or there is no standard treatment scheme, or standard treatment is not applicable at this stage.

In some embodiments, the tumor is selected from breast cancer, gastric cancer, lung cancer, ovarian cancer, urothelial cancer, esophageal cancer, liver cancer, colorectal cancer, cervical cancer, endometrial cancer, pancreatic cancer, bladder cancer, or brain tumor; and preferably breast cancer (e.g. triple-negative breast cancer or Her2-positive breast cancer), ovarian cancer (e.g. ovarian epithelial cancer), gastric cancer, lung cancer, pancreatic cancer, bladder cancer, or urothelial cancer; and more preferably, the tumor disease is triple-negative breast cancer, Her2-positive breast cancer, ovarian cancer, gastric cancer, lung cancer or pancreatic cancer.

In some embodiments, the tumor is triple-negative breast cancer, Her2-positive breast cancer, ovarian cancer or gastric cancer.

### BRIEF DESCRIPTION OF THE DRAWINGS

The drawings described herein are used for providing further understanding of the present invention and constitute a part of the present application. The exemplary embodiments of the present invention and descriptions thereof are used for explaining the present invention, and do not constitute improper limitations on the present invention. In the accompanying drawings:

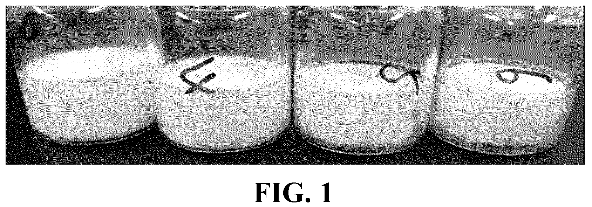

FIG. 1: appearance of a lyophilized product in Experimental Example 4: in the figure, a composition No. 12 is contained in a vial No. 4, a composition No. 13 is contained in a vial No. 5, and a composition No. 14 is contained in a vial No. 6.

### DETAILED DESCRIPTION

The technical solutions in the embodiments of the present invention will be described clearly and completely below in conjunction with the drawings in the embodiments of the present invention. Obviously, the described embodiments are only part of the embodiments of the present invention, rather than all the embodiments. The following description of at least one exemplary embodiment is merely illustrative in nature and is in no way intended to limit the present invention, its application, or uses. All other embodiments obtained by those of ordinary skills in the art based on the embodiments of the present invention without creative efforts are within the claimed scope of the present invention.

Unless otherwise defined hereinafter, all technical and scientific terms as used herein have the same meaning as commonly understood by those skilled in the art. The terms used in the specification of the present invention are only for the purpose of describing specific embodiments, and are not intended to limit the present invention. As used herein, the term "and/or" includes any and all combinations of one or more related listed items.

In the examples, the observation results of the appearance of the pharmaceutical composition are represented by codes, and the meanings of the codes are as follows:
level 1: clear and without visible protein particles (qualified);
level 2: a. clear but with visible fine protein particles (the number of particles < 10, < 100 um); and b. slightly opalescent but with non-obvious protein particles; (qualified);
level 3: a. the number of visible protein particles is greater than 10 (< 100 um) (unqualified); b. a trace of protein flocs are visible (> 150 um); and c. slightly turbid or misty (unqualified);
level 4: moderate turbid (unqualified); and
level 5: deeply turbid, white and milky (unqualified).

Abbreviations in the present invention have the following meanings:

| Abbreviation | Meaning | Abbreviation | Meaning |
|---|---|---|---|
| PB/PBS | Phosphate buffer | DAR | Drug-to-antibody ratio |
| SEC-HPLC | Size Exclusion Chromatography | L-1 | Antibody chain conjugated to 1 drug molecule |
| H-3 | Antibody heavy chain conjugated to 3 drug molecules | NDLC | Antibody light chain conjugated to no drug |
| NDHC | Antibody heavy chain conjugated to no drug | \ | \ |

### Example 1 preparation of 4-((S)-2-(4-aminobutyl)-35-(4-((6-(2-(methylsulfonyl)pyrimidin-5-yl)hex-5-amido)methyl)-1H-1,2,3-triazol-1-yl)-4,8-dioxo-6,12,15,18,21,24,27,30,33-nonaoxa-3,9-diazapentatriacontylamido)benzyl ((S)-4-ethyl-11-(2-(N-isopropylmethylsulfonamido)ethyl)-3,14-dioxo-3,4,12,14-tetrahydro-1H-pyrano[3',4':6,7]indolizino[1,2-b]quinolin-4-yl)carbonate

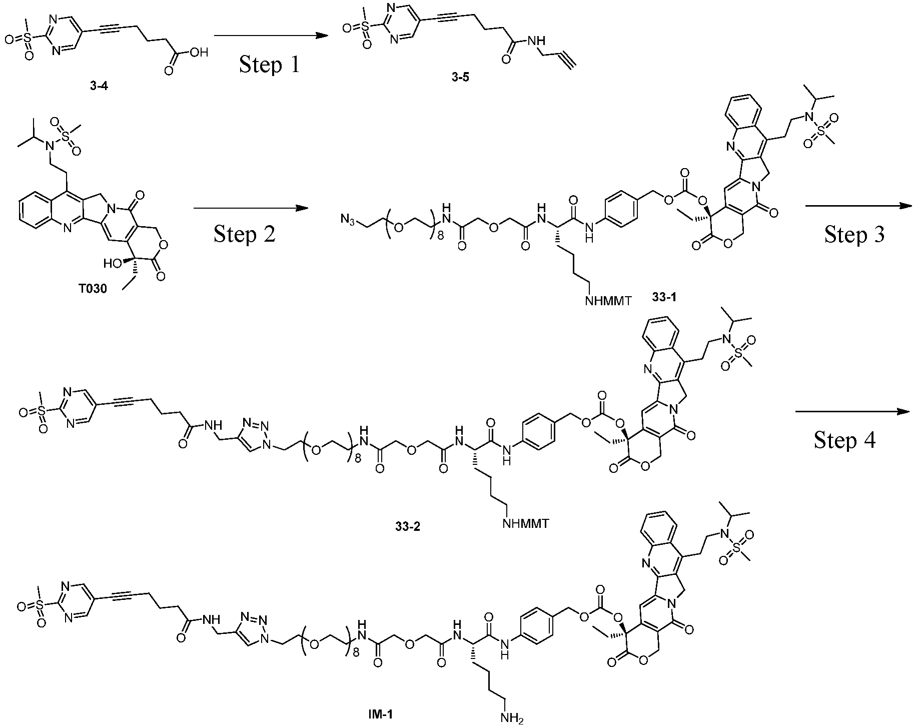

### Step 1: synthesis of 6-(2-(methylsulfonyl)pyrimidin-5-yl)-N-(prop-2-yn-1-yl)hex-5-ynamide

At 25°C, prop-2-yn-1-amine (189 mg, 3.4 mmol) and a compound 3-4 (800 mg, 2.83 mmol) were dissolved in dichloromethane (10 mL), sequentially added with N,N-diisopropylethylamine (738 mg, 5.67 mmol), O-(7-azabenzotriazole-1-yl)-N,N,N',N'-tetramethylureahexafluorophosphate (1.63 g, 4.25 mmol), and stirred for reaction for 2 h. The reaction solution was concentrated under reduced pressure, and the residue was purified by a rapid silica gel column (ethyl acetate/petroleum ether = 3/1) to obtain 700 mg of the title compound. ESI-MS (m/z): 306.1[M+H]+.

### Step 2: synthesis of 4-((S)-35-azido-2-(4-(((4-methoxyphenyl)benzhydryl)amino)butyl)-4,8-dioxo-6,12,15,18,21,24,27,30,33-nonaoxa-3,9-diazapentatriacontylamido)benzyl ((S)-4-ethyl-11-(2-(N-isopropylmethylsulfonamido)ethyl)-3,14-dioxo-3,4,12,14-tetrahydro-2H-pyrano[2,3-b]-1H-pyrano[3',4':6,7]indolizino[1,2-b]quinolin-4-yl)carbonate

Under nitrogen protection at 25°C, T-030 (250 mg, 0.49 mmol) was dissolved in dichloromethane (10 mL), cooled to 0°C, added with a solution of 4-dimethylaminopyridine (478 mg, 3.91 mmol) in dichloromethane (3 mL), then slowly added dropwise with a solution of triphosgene (72 mg, 0.24 mmol) in dichloromethane (10 mL), and then stirred at 0°C for reaction for 20 min. The reaction solution was purged with nitrogen for 20 min. The reaction solution was added with a solution of (S)-2-(32-azido-5-oxo-3,9,12,15,18,21,24,27,30-nonaoxa-6-azatriacetamido)-N-(4-(hydroxymethyl)phenyl)-6(((4-methoxyphenyl)benzhydryl)amino)acetamide (518 mg, 0.49 mmol) in dichloromethane (7 mL), and then stirred at 0°C for reaction for 1 h. The reaction solution was concentrated under reduced pressure, and the residue was purified by preparative high performance liquid chromatography to obtain 500 mg of the title compound. ESI-MS (m/z): 1597.5[M+H]+.

### Step 3: synthesis of (S)-4-ethyl-11-(2-(N-isopropylmethylsulfonamido)ethyl)-3,14-dioxo-3,4,12,14-tetrahydro-1H-pyrano[3',4':6,7]indolizino[1,2-b]quinolin-4-yl (4-((S)-2-(4-(((4-methoxyphenyl)diphenylmethyl)amino)butyl)-35-(4-((6-(2-(methylsulfonyl)pyrimidin-5-yl)hex-5-amido)methyl)-1H-1,2,3-triazol-1-yl)-4,8-dioxo-6,12,15,18,21,24,27,30,33-nonaoxa-3,9-diazapentatriacontylamido)benzyl)carbonate

At room temperature, a compound 33-1 (14 mg, 0.05 mmol) was dissolved in dimethyl sulfoxide and water (2.0 mL: 0.5 mL), added with cuprous bromide (11 mg, 0.08 mmol), and stirred for reaction for 1 h. The product was purified by preparative high performance liquid chromatography to obtain 30 mg of the title compound. ESI-MS (m/z): 815.9[(M-273)/2+H]+.

### Step 4: synthesis of 4-((S)-2-(4-aminobutyl)-35-(4-((6-(2-(methylsulfonyl)pyrimidin-5-yl)hex-5-amido)methyl)-1H-1,2,3-triazol-1-yl)-4,8-dioxo-6,12,15,18,21,24,27,30,33-nonaoxa-3,9-diazapentatriacontylamido)benzyl ((S)-4-ethyl-11-(2-(N-isopropylmethylsulfonamido)ethyl)-3,14-dioxo-3,4,12,14-tetrahydro-1H-pyrano[3',4':6,7]indolizino[1,2-b]quinolin-4-yl)carbonate (compound IM-1)

A compound 33-2 (30 mg, 0.02 mmol) was dissolved in dichloromethane (1.0 mL), and the reaction solution was added with trifluoroacetic acid (0.2 mL) to react at room temperature for 30 min. The product was purified by preparative high performance liquid chromatography (method C) to obtain 20.0 mg of a trifluoroacetate of the title compound. The structural characterization of it was as follows:
¹H NMR (400 MHz, DMSO-d6) δ 10.18 (s, 1H), 9.10 (s, 2H), 8.38 (t, J = 5.56 Hz, 1H), 8.32 (d, J = 8.40 Hz, 1H), 8.22 - 8.20 (m, 2H), 8.09 (t, J = 5.68 Hz, 1H), 7.91-7.87 (m, 2H), 7.82-7.78 (m, 1H), 7.69 (brs, 3H), 7.61 (d, J = 8.56 Hz, 2H), 7.32 (d, J = 8.56 Hz, 2H), 7.06 (s, 1H), 5.56 (d, J = 19.28 Hz, 1H), 5.14 (d, J = 12.20 Hz, 1H), 5.07 (d, J = 12.16 Hz, 1H), 4.48 (t, J = 5.24 Hz, 2H), 4.46 - 4.43 (m, 1H), 4.29 (d, J = 5.60 Hz, 2H), 4.08 - 3.95 (m, 5H), 3.79 (t, J = 5.28 Hz, 2H), 3.51 - 3.43 (m, 32H), 3.40 (s, 3H), 3.39 - 3.35 (m, 2H), 3.30 - 3.26 (m, 2H), 3.00 (s, 3H), 2.82 - 2.74 (m, 2H), 2.56 (t, J = 7.08 Hz, 2H), 2.29 (t, J = 7.36 Hz, 2H), 2.23 - 2.13 (m, 2H), 1.82 (p, J = 7.24 Hz, 2H), 1.78 - 1.63 (m, 2H), 1.61 - 1.49 (m, 2H), 1.42 - 1.27 (m, 2H), 1.15 (d, J = 6.80 Hz, 3H), 1.13 (d, J = 6.76 Hz, 3H) , 0.90 (t, J = 7.32 Hz, 3H). ESI-MS (m/z): 816.0[M/2⁺H]⁺. [α]_{D}²⁰ is - 19.55° (c = 1.000 g/100 mL, CH₃CN).

### Example 2. Preparation of conjugate A

0.3 mL of a Sacituzumab antibody (anti-Trop-2, 33.5 mg/mL) was taken, diluted with 0.25 mL of a solution (pH 7.6) containing 20 mM PB, 150 mM NaCl and 20 mM sodium edetate, then added with 0.45 mL of a solution containing 20 mM PB and 150 mM NaCl (pH 7.6) and mixed well, adjusted to the pH of 7.4 with a 1 M Na₂HPO₄ solution, added with a 10 mM TCEP (tris(2-carboxyethyl)phosphine) solution, mixed well, and allowed to stand at room temperature for 30 min. The aforementioned solution system was added with 10 times the amount of an IM-1 trifluoroacetate dissolved in dimethyl sulfoxide, mixed well, allowed to stand at room temperature for 2 h, and then added with 6.1 µl of 100 mM cysteine to terminate the reaction. Finally, the buffer solution was replaced with a PBS buffer solution at pH 6.5 by utilizing a G-25 gel column to obtain a product of IM-1 conjugated with the Sacituzumab antibody, which was named a conjugate A.

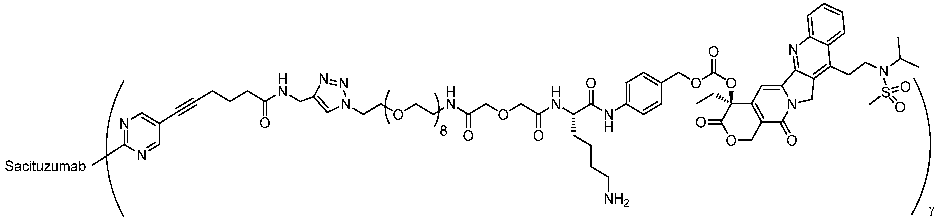

The molecular weight of the conjugate A was analyzed by a LCMS method. The measured molecular weights of the light and heavy chains of the conjugate A were correlated with the theoretical molecular weights of the light and heavy chains conjugated with different numbers of toxins. It was determined that 1-10 toxins were conjugated to each antibody molecule in the conjugate A (i.e. γ was 1-10). Therefore, the average drug-to-antibody ratio (DAR) of approximately 6.9 was calculated according to the percentages of conjugate molecules conjugated with different numbers of toxins.

Several batches of preparation were conducted by a similar process to obtain conjugate A samples with DAR values ranging from 6-8 for the following preparation studies.

### Experimental Example 1 Effect of buffer system on stability

Each ingredient was formulated into pharmaceutical compositions Nos. 1-4 with water for injection. The type of each ingredient and its concentration or mass volume percentage (g/100 ml) in the pharmaceutical compositions were as shown in Table 1. The changes in the ingredients of each pharmaceutical composition at an initial state and after being placed under conditions of 5°C, 25°C, or 40°C respectively for 14 days were observed or detected. The detection results were as shown in Table 2:

**Table 1**

| Composition No. | Composition ingredients and concentrations thereof | pH |
|---|---|---|
| 1 | 12.5 mg/ml of the conjugate A + 10 mM histidine/histidine hydrochloride + 0.03% of polysorbate 20 + 4% of sucrose | 6.0 |
| 2 | 12.5 mg/ml of the conjugate A + 10 mM histidine/histidine hydrochloride + 0.03% of polysorbate 20 + 4% of sucrose | 6.5 |
| 3 | 7.6 mg/ml of the conjugate A + 10 mM citric acid + 0.03% of polysorbate 20 + 4% of sucrose | 6.0 |
| 4 | 12.5 mg/ml of the conjugate A + 10 mM PBS + 0.01% of polysorbate 80 + 6% of sucrose | 6.5 |

**Table 2**

| Composition No. | Inspection conditions | SEC-HPLC | |
|---|---|---|---|
| | | Aggregate | Main peak |
| 1 | Day 0 | 1.2 | 98.8 |
| | 40°C | 1.5 | 96.8 |
| | 25°C | 0.93 | 98.8 |
| | 5°C | 1.67 | 98.1 |
| 2 | Day 0 | 1.1 | 98.9 |
| | 25°C | 1.22 | 98.5 |
| 3 | Day 0 | 1.5 | 98.5 |
| | 40°C | 4.7 | 94.9 |
| | 25°C | 2.31 | 97.4 |
| | 5°C | 2.36 | 97.5 |
| 4 | Day 0 | 2.2 | 97.8 |
| | 25°C | 4.05 | 96.8 |

According to the aforementioned determination results, it could be seen that after the compositions Nos. 1 and 2 using histidine/histidine hydrochloride as the buffer system were placed under several temperature conditions of 40°C, 25°C and 5°C for fourteen days, the purity of the active ingredients in the samples did not decrease substantially, and the amount of the produced aggregate impurities was significantly lower than those of the compositions Nos. 3 and 4. It could be seen that the histidine/histidine hydrochloride buffer system help to improve the stability of the pharmaceutical composition of the present invention.

### Experimental Example 2 Effect of pH conditions on stability of composition

Each ingredient was formulated into pharmaceutical compositions Nos. 5-8 with water for injection. The type of each ingredient and its concentration or mass volume percentage (g/100 ml) in the pharmaceutical compositions were as shown in Table 3. The changes in the appearance and ingredients of each pharmaceutical composition at an initial state and after being placed under conditions of 5°C respectively for 14 days were observed or detected. The detection results were as shown in Table 4:

**Table 3**

| Composition No. | Composition ingredients and weight percentages thereof | pH |
|---|---|---|
| 5 | 20 mg/ml of the conjugate A + 10 mM histidine/histidine hydrochloride + 0.02% of polysorbate 20 + 3% of sucrose + 3% mannitol | 5.4 |
| 6 | 20 mg/ml of the conjugate A + 10 mM histidine/histidine hydrochloride + 0.02% of polysorbate 20 + 3% of sucrose + 3% mannitol | 5.7 |
| 7 | 20 mg/ml of the conjugate A + 10 mM histidine/histidine hydrochloride + 0.02% of polysorbate 20 + 3% of sucrose + 3% mannitol | 6.3 |
| 8 | 20 mg/ml of the conjugate A + 10 mM histidine/histidine hydrochloride + 0.02% of polysorbate 20 + 3% of sucrose + 3% mannitol | 6.0 |

**Table 4**

| Composition No. | Placement time | Appearan ce | SEC-HPLC | | |
|---|---|---|---|---|---|
| | | | Aggregate (%) | Main peak (%) | Small-molecule impurities (%) |
| 5 | Day 0 | 1 | 2.6 | 97.3 | 0.1 |
| | Day 8 | 2a | 2.3 | 97.5 | 0.1 |
| | Day 14 | 2a | 2.6 | 97.3 | 0.1 |
| 6 | Day 0 | 1 | 2.6 | 97.4 | 0.1 |
| | Day 8 | 1 | 2.4 | 97.5 | 0.1 |
| | Day 14 | 2a | 2.6 | 97.3 | 0.1 |
| 7 | Day 0 | 1 | 2.6 | 97.3 | 0.1 |
| | Day 8 | 1 | 2.4 | 97.5 | 0.2 |
| | Day 14 | 2a | 2.5 | 97.4 | 0.1 |
| 8 | Day 0 | 1 | 2.6 | 97.3 | 0.1 |
| | Day 8 | 1 | 2.4 | 97.5 | 0.1 |
| | Day 14 | 2a | 2.6 | 97.3 | 0.1 |

It could be seen from the aforementioned determination results that when the pH was in the range of 5.4-6.3, the compositions all had good stability, where when the pH was in the range of 5.7-6.3, a state in which the appearance of the composition remained clear and there were no visible protein particles could be maintained for more than eight days, and thus the composition had better stability.

### Experimental Example 3 Effect of ADC concentration on stability

Each ingredient was formulated into pharmaceutical compositions Nos. 9-11 with water for injection. The type of each ingredient and its concentration or mass volume percentage (g/100 ml) in the pharmaceutical compositions were as shown in Table 5. The changes in the appearance and ingredients of each pharmaceutical composition at an initial state and after being placed under conditions of 25°C for 14 days were observed or detected. The detection results were as shown in Table 6:

**Table 5**

| Composition No. | pH value | Concentration of conjugate A | Component | | | |
|---|---|---|---|---|---|---|
| | | | Histidine/histidine hydrochloride | Polysorbate-20 | Sucrose | Mannitol |
| 9 | 6.0 | 20 mg/ml | 10 mM | 0.02% | 3% | 3% |
| 10 | 6.0 | 30 mg/ml | 10 mM | 0.02% | 3% | 3% |
| 11 | 6.0 | 40 mg/ml | 10 mM | 0.02% | 3% | 3% |

**Table 6**

| Composition No. | Placement time | Appearance | SEC-HPLC | | |
|---|---|---|---|---|---|
| | | | Aggregate (%) | Main peak (%) | Small-molecule impurities (%) |
| 9 | Day 0 | 1 | 2.6 | 97.3 | 0.1 |
| | Day 4 | 1 | 2.2 | 97.7 | 0.1 |
| | Day 14 | Proteins precipitated and adhered to the wall, and there were suspended particles in the solution | 2.4 | 97.4 | 0.2 |
| 10 | Day 0 | 1 | 2.6 | 97.3 | 0.1 |
| | Day 4 | 2a | 2.4 | 97.5 | 0.1 |
| | Day 14 | Proteins precipitated and adhered to the wall, and there were suspended particles in the solution | 2.8 | 97.1 | 0.2 |
| 11 | Day 0 | 1 | 2.7 | 97.2 | 0.1 |
| | Day 4 | 1 | 2.6 | 97.3 | 0.1 |
| | Day 14 | Proteins precipitated and adhered to the wall, and there were suspended particles in the solution | 3.1 | 96.8 | 0.2 |

It could be seen from the aforementioned results that when the ADC concentration was 20 mg/ml, the pharmaceutical composition had the best stability.

### Experimental Example 4 Effect of component content on stability

Pharmaceutical compositions Nos. 12-14 were formulated as shown in Table 7 below and lyophilized, and the changes in the appearance and ingredients of each pharmaceutical composition at the initial state and after being placed under a condition of 25°C for 14 days were observed or detected. The detection results were as shown in Table 8:

**Table 7**

| Composition No. | pH value | ADC Concentration | Component | | | |
|---|---|---|---|---|---|---|
| | | | Histidine/histidine hydrochloride | Polysorbate-20 | Sucrose | Mannitol |
| 12 | 6.0 | 20 mg/ml | 10 mM | 0.02% | 3% | 3% |
| 13 | 6.0 | 20 mg/ml | 10 mM | 0.02% | 6% | 1.5% |
| 14 | 6.0 | 20 mg/ml | 10 mM | 0.02% | 8% | 0% |

**Table 8**

| Composition No. | Placement time | Appearance | SEC-HPLC | | |
|---|---|---|---|---|---|
| | | | Aggregate (%) | Main peak (%) | Small-molecule impurities (%) |
| 12 | Day 0 | 1 | 2.6 | 97.3 | 0.1 |
| | Day 8 | 1 | 2.2 | 97.7 | 0.1 |
| | Day 14 | Proteins precipitated and adhered to the wall, and there were suspended particles in the solution | 2.4 | 97.4 | 0.2 |
| 13 | Day 0 | 1 | 2.6 | 97.4 | 0.1 |
| | Day 8 | 2a | 2.2 | 97.7 | 0.1 |
| | Day 14 | Proteins precipitated and adhered to the wall, and there were suspended particles in the solution | 2.5 | 97.4 | 0.2 |
| 14 | Day 0 | 1 | 2.6 | 97.3 | 0.1 |
| | Day 8 | 1 | 2.1 | 97.7 | 0.1 |
| | Day 14 | Proteins precipitated and adhered to the wall, and there were suspended particles in the solution | 2.5 | 97.4 | 0.2 |

It could be seen from the aforementioned determination results that the compositions formulated by adjusting the content of each auxiliary material within a certain range all had good stability. Combined with the appearance of the pharmaceutical composition shown in FIG. 1, compositions 12 and 14 maintained better appearances and better stability after lyophilization.

### Experimental Example 5 Effect of components on stability of composition

Pharmaceutical compositions Nos. 15 and 16 were formulated as shown in the Table 9 below and lyophilized. The lyophilizing procedure included feeding; pre-freezing under a condition of 0 - -50°C for 400-800 minutes; evacuating to a vacuum degree of 20 Pa; subliming at a temperature range of -30-0°C and a pressure of 0-100 pa for a time period of 2,000-5,000 minutes; and then conducting desorption drying at a temperature range of 0 - -50°C and a pressure of 0-100 pa for a time period of 200-2,000 minutes. The changes in the ingredient contents of each pharmaceutical composition at the initial state, before and after lyophilization, and after being placed under a condition of 40°C for 0.5-4 months were observed or detected. The detection results were as shown in Table 10:

**Table 9**

| Serial number | ADC Concentration | Buffer system (Histidine/histidine hydrochloride) | Auxiliary materials | | |
|---|---|---|---|---|---|
| | | | Sucros e | Mannito l | Tween-20 (%) |
| 15 | 20 mg/ml | 10 mM | 3% | 3% | 0.02 |
| 16 | 20 mg/ml | 10 mM | 8% | / | 0.02 |

**Table 10**

| Composition No. | Placement time | Concentration of free toxin (µg/ml) | SEC-HPLC | | |
|---|---|---|---|---|---|
| | | | Aggregate (%) | Main peak (%) | Small-molecule impurities (%) |
| 15 | Day 0 | 8.36 | 1.6 | 98.2 | 0.2 |
| | 0 days after lyophilization | 10.37 | 1.8 | 98.0 | 0.2 |
| | 0.5 months after lyophilization | 19.69 | 2.49 | 97.4 | 0.09 |
| | 1 months after lyophilization | 23.42 | 2.8 | 97.1 | 0.2 |
| | 2 months after lyophilization | 20.95 | 3.5 | 96.3 | 0.2 |
| | 4 months after lyophilization | 40.81 | 3.0 | 97.0 | / |
| 16 | Day 0 | 8.35 | 1.6 | 98.3 | 0.2 |
| | 0 days after lyophilization | 9.42 | 1.7 | 98.2 | 0.2 |
| | 0.5 months after lyophilization | 13.98 | 1.66 | 98.22 | 0.12 |
| | 1 months after lyophilization | 14.08 | 1.8 | 98.1 | 0.1 |
| | 2 months after lyophilization | 14.31 | 2.1 | 97.8 | 0.1 |
| | 4 months after lyophilization | 19.63 | 1.46 | 98.54 | / |

According to the aforementioned results, it could be seen that for the composition No. 16 without mannitol, after storage for 4 months, the impurity ingredients such as aggregates, small-molecule impurities, free toxins, etc. were increased less, and the content of active ingredients remained basically unchanged, so it had better stability.

### Experimental Example 6 Improvement of stability of antibody-drug conjugate by lyophilized composition

### (1) Stability of antibody-drug conjugates before lyophilization

A liquid pharmaceutical composition was formulated according to the following ingredients: 20 g/L of the antibody-drug conjugate A, 0.73 g/L of histidine, 1.11 g/L of histidine hydrochloride, at a pH value of 6.0, 0.02% (w/v) of polysorbate 20, and 80 g/L of sucrose. The pharmaceutical composition was stored under a condition of 5 ± 3°C for 3 months and under a condition of 25°C for 7 days. During this period, changes in its appearance, the content of each ingredient, etc. were observed. The observation results were as shown in Tables 11 and 12.

**Table 11 Stability of conjugate A before lyophilization under condition of 5 ± 3°C**

| Detection Items | | Acceptance criteria | 0 month | 1 months | 2 months | 3 months |
|---|---|---|---|---|---|---|
| Clarity¹ | | ≤ standard solution No. 3 | < standard solution No. 2 | NT | NT | < standard solution No. 4 |
| Drug load | L-1 | ≥ 85.0% | 96.4 | 93.5 | 91.5 | 89.4 |
| | H-3 | ≥ 60.0% | 77.7 | 68.8 | 66.8 | 64.7 |
| SEC-HPLC | Main peak | ≥ 95.0% | 99.0 | 99.8 | 99.8 | 99.7 |
| | Aggregate | ≤ 3.0% | 0.1 | 0.2 | 0.2 | 0.3 |
| | Small molecule | ≤ 2.0% | 0.0 | 0.0 | 0.0 | 0.1 |
| Free toxin | | ≤ 8.4% | 0.6 | 4.7 | 7.2 | 11.5 |
| Free antibody | NDLC | ≤ 10.0% | 3.6 | 6.5 | 8.5 | 10.6 |
| | NDHC | ≤ 5.0% | 0.5 | 0.8 | 0.9 | 0.8 |

| | | | | | | |
|---|---|---|---|---|---|---|
| ¹ referred to that the clarity test specified in the Chinese Pharmacopoeia was conducted and the clarity was compared with that of a turbidity standard solution specified in the Chinese Pharmacopoeia. | | | | | | |

As could be seen from the table above, the free toxins in the antibody-drug conjugate before lyophilization increased significantly after placement for 1 month, and exceeded a quality standard range after placement for 3 months.

**Table 12 Stability of conjugate A before lyophilization under condition of 25°C**

| Detection Items | | Acceptance criteria | Day 0 | High temperature of 25°C | | | |
|---|---|---|---|---|---|---|---|
| | | | | 4 hours | Day 1 | Day 3 | Day 7 |
| DAR | | 6.7-7.7 | 7.4 | 7.4 | 7.2 | 6.9 | 6.6 |
| Drug load | L-1 | ≥ 85.0% | 96.4 | 96.4 | 93.9 | 89.8 | 85.9 |
| | H-3 | ≥ 60.0% | 77.7 | 77 | 72 | 63.8 | 56.8 |
| SEC-HPLC | Main peak | ≥ 95.0% | 99.9 | 99.9 | 99.8 | 99.8 | 99.8 |
| | Aggreg ate | ≤ 3.0% | 0.1 | 0.1 | 0.2 | 0.2 | 0.2 |
| | Small molecul e | ≤ 2.0% | 0.0 | 0.0 | 0.0 | 0.0 | 0.0 |
| Free toxin | | ≤ 8.4% (mol/mol) | 0.6 | 1.3 | 4.7 | 9.9 | 16.1 |
| Free antibody | NDLC | ≤ 10.0% | 3.6 | 3.6 | 6.1 | 10.2 | 14.1 |
| | NDHC | ≤ 5.0% | 0.5 | 0.5 | 0.4 | 0.7 | 1.3 |

As could be seen from the above table, when the antibody-drug conjugate before lyophilization was placed under a condition of 25°C for 4 hours, the free toxin was increased slightly. After placement for 1 day, the DAR value and drug load were decreased significantly, and the free antibody was increased significantly. When the placement time was extended to 3 days, the free toxin exceeded the quality standard range and the stability was poor.

### (2) Stability of lyophilized products

A solution was formulated according to the following ingredients and lyophilized in a manner similar to that of Experimental Example 5: 20 mg/ml of the antibody-drug conjugate A, 0.73 g/L of histidine, 1.11 g/L of histidine hydrochloride, at a pH value of 6.0, 0.02% (w/v) of polysorbate 20, and 80 g/L of sucrose. The samples were stored under conditions of 5°C, 25 ± 2°C and 40°C respectively. During this period, changes in their appearances, the content of each ingredient, etc. were observed. The observation results were as shown in Tables 13-15.

**Table 13 Stability of lyophilized products under condition of 5°C**

| Detection Items | | Acceptance criteria | 0 month | 6 months | 12 months | 18 months | 24 months |
|---|---|---|---|---|---|---|---|
| DAR | | 6.7-7.7 | 7.2 | 7.3 | 7.3 | 7.2 | 7.3 |
| SEC-HPLC | Main peak | ≥ 95.0% | 99.7 | 99.8 | 99.8 | 99.7 | 99.8 |
| | Aggregate | ≤ 3.0% | 0.2 | 0.2 | 0.2 | 0.2 | 0.2 |
| | Small molecule | ≤ 2.0% | 0.1 | 0.0 | 0.0 | 0.0 | 0.0 |
| Drug load | L-1 | ≥ 85.0% | 95.7 | 95.6 | 95.7 | 95.5 | 95.6 |
| | H-3 | ≥ 60.0% | 72.1 | 76.0 | 73.7 | 71.4 | 72.8 |
| Free toxin | | ≤ 8.4% (mol/mol) | 0.7 | 1.9 | 0.9 | 1.0 | 1.1 |
| Free antibody | NDLC | ≤ 10.0% | 4.3 | 4.4 | 4.3 | 4.5 | 4.4 |
| | NDHC | ≤ 5.0% | 0.8 | 0.3 | 0.7 | 0.7 | 0.6 |

As shown in the table above, the lyophilized product was placed at 5°C for 24 months without significant changes in sample quality, and its stability was significantly improved compared with that of the product before lyophilization.

**Table 14 Stability of lyophilized products under condition of 25 ± 2°C**

| Detection Items | | Acceptance criteria | 0 month | 3 months | 6 months | 12 months |
|---|---|---|---|---|---|---|
| DAR | | 6.7-7.7 | 7.2 | 7.3 | 7.3 | 7.3 |
| SEC-HPLC | Main peak | ≥ 95.0% | 99.7 | 99.6 | 99.7 | 99.6 |
| | Aggregate | ≤ 3.0% | 0.2 | 0.3 | 0.3 | 0.4 |
| | Small molecule | ≤ 2.0% | 0.1 | 0.0 | 0.0 | 0.0 |
| Drug load | L-1 | ≥ 85.0% | 95.7 | 95.9 | 95.4 | 95.5 |
| | H-3 | ≥ 60.0% | 72.1 | 74.5 | 75.8 | 73.4 |
| Free toxin | | ≤ 8.4% (mol/mol) | 0.7 | 0.8 | 2.1 | 1.2 |
| Free antibody | NDLC | ≤ 10.0% | 4.3 | 4.1 | 4.6 | 4.5 |
| | NDHC | ≤ 5.0% | 0.8 | 0.6 | 0.3 | 0.7 |

As shown in the table above, the lyophilized product was placed at 25°C for 12 months, and the detection results did not change significantly, which met the quality standard requirements. Compared with the product before lyophilization, the stability was significantly improved.

**Table 15 Stability of lyophilized products under condition of 40°C**

| Detection Items | | Acceptance criteria | 0 month | High temperature of 40 ± 2°C | |
|---|---|---|---|---|---|
| | | | | Day 7 | Day 14 |
| DAR | | 6.7-7.7 | 7.4 | 7.3 | 7.4 |
| SEC-HPLC | Main peak | ≥ 95.0% | 99.8 | 99.8 | 99.8 |
| | Aggregate | ≤ 3.0% | 0.2 | 0.2 | 0.2 |
| | Small molecule | ≤ 2.0% | 0.0 | 0.0 | 0.0 |
| Drug load | L-1 | ≥ 85.0% | 95.7 | 95.5 | 96.5 |
| | H-3 | ≥ 60.0% | 77.0 | 75.5 | 76.7 |
| Free toxin | | ≤ 8.4% (mol/mol) | 1.2 | 2.4 | 1.2 |
| Free antibody | NDLC | ≤ 10.0% | 4.0 | 4.5 | 3.5 |
| | NDHC | ≤ 5.0% | 0.3 | 0.3 | 0.5 |

As shown in the table above, the lyophilized product was placed under a condition of 40°C for 14 consecutive days without affecting the quality of the sample.

### Example 7. Reconstitution stability

A solution was formulated according to the following ingredients and lyophilized in a manner similar to that of Experimental Example 5: 20 mg/ml of the antibody-drug conjugate A, 0.73 g/L of histidine, 1.11 g/L of histidine hydrochloride, at a pH value of 6.0, 0.02% (w/v) of polysorbate 20, and 80 g/L of sucrose. After reconstitution with sterile water for injection, the solution was stored under conditions of 5°C and 25°C for 1 day, 3 days or 7 days, respectively, and the changes in the content of each ingredient of it were determined. The results were as shown in Table 16.

**Table 16**

| Sampling point | Free toxin (%mol/mol) | DAR value | SEC-HPLC | |
|---|---|---|---|---|
| | | | Aggregate (%) | Main peak (%) |
| Day 0 | 2.1 | 7.4 | 0.1 | 99.9 |
| 5°C for 1 day | 2.4 | 7.4 | 0.2 | 99.8 |
| 5°C for 3 days | 2.6 | 7.4 | 0.1 | 99.9 |
| 5°C for 7 days | 2.9 | 7.4 | 0.2 | 99.8 |
| 25°C for 1 day | 4.9 | 7.3 | 0.2 | 99.8 |
| 25°C for 3 days | 9.1 | 7.0 | 0.2 | 99.8 |
| 25°C for 7 days | 14.7 | 6.7 | 0.2 | 99.8 |

As could be seen in the table above, after the lyophilized product of antibody-drug conjugate was reconstituted with sterile water for injection, it could be stored at 5 ± 3°C for 7 days and at 25 ± 2°C for 1 day, which met the clinical medication needs.

The examples described above are merely illustrative of several embodiments of the present invention, the description of them is more specific and detailed, but cannot be construed as limiting the scope of the present invention accordingly. It should be noted that, several variations and modifications can be made by those of ordinary skills in the art, under the premise of not departing from the concept of the present invention, and these variations and modifications all fall within the claimed scope of the present invention. Therefore, the claimed scope of the patent of the present invention shall be determined by the appended claims.

## Claims

1. A pharmaceutical composition, comprising the following components:
an antibody-drug conjugate, a buffer system, a lyoprotectant, and a surfactant.

2. The pharmaceutical composition according to claim 1, **characterized by** one or more of the following:
(1) the buffer system is selected from one or more of histidine hydrochloride/histidine, succinic acid/sodium succinate, and citric acid/sodium citrate;
(2) a concentration range of the buffer system in the pharmaceutical composition is 5-50 mM;
(3) the lyoprotectant is selected from one or more of sucrose, trehalose, sorbitol, and mannitol; and
(4) the surfactant is selected from polysorbate or poloxamer.

3. The pharmaceutical composition according to claim 1 or 2, wherein the buffer system is histidine hydrochloride/histidine.

4. The pharmaceutical composition according to any one of claims 1-3, wherein the lyoprotectant is sucrose.

5. The pharmaceutical composition according to any one of claims 1-4, wherein the surfactant is polysorbate 20 or polysorbate 80.

6. The pharmaceutical composition according to any one of claims 1-4, wherein the surfactant is polysorbate 20.

7. The pharmaceutical composition according to any one of claims 1-6, wherein the antibody-drug conjugate has a structure of formula (I):
{D-[L₁-(L₂)m₁-(L₃)m₂-(L₄)m₃-E]}_{γ}-A formula (I)
wherein
L₁ is 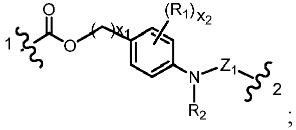 R₁ and R₂ are each independently hydrogen (e.g., protium or deuterium), halogen, carboxylic acid, sulfonic acid, cyano, C₁₋₆ alkyl, halogenated C₁₋₆ alkyl, cyano-substituted C₁₋₆ alkyl (e.g., -CH₂CN), C₁₋₆ alkoxy, C₂₋₁₀alkenyl or C₂₋₁₀ alkynyl; Z₁ is an amino acid or a peptide composed of 2-10 amino acids; x₁ and x₂ are each independently 0, 1, 2, 3, 4, 5 or 6; and the position 1 of L₁ is linked to D, and the position 2 of L₁ is linked to L₂;
L₂ is 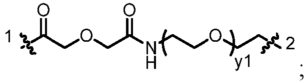 wherein yi is 0, 1, 2, 3, 4, 5, 6, 7, 8, 9 or 10; and the position 1 of L₂ is linked to the L₁, and the position 2 of L₂ is linked to L₃;
L₃ is a 5-12 membered heteroaromatic ring;
L4 is 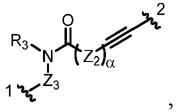 wherein Z₂ is selected from C₁₋₆ alkylene, C₂₋₁₀ alkenylene, C₂₋₁₀ alkynylene, and C₃₋₈ cycloalkylene; R₃ is selected from H and C₁₋₆ alkyl; Z₃ is absent or C₁₋₆ alkylene; or R₃ and Z₃, together with a nitrogen atom to which they are attached, form a 4-8 membered heterocyclic group; α is 0, 1, 2, 3, 4, 5 or 6, and the position 2 of L₄ is linked to E, and the position 1 of L₄ is linked to L₃;
E is 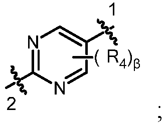 wherein each R₄ is independently hydrogen (e.g., protium or deuterium), β is 0, 1 or 2, and the position 2 of E is linked to A, and the position 1 of E is linked to L₄;
mi, m₂ and m₃ are each independently 0, 1, 2, 3, 4, 5, 6, 7, 8, 9 or 10;
D is a bioactive molecular fragment;
γ refers to the number of {D-[L₁-(L₂)ₘ₁-(L₃)ₘ₂-(L₄)ₘ₃-E]} moieties that form a thioether bond with the sulfhydryl group of A, which is selected from 1-10;
A is an anti-Trop-2 monoclonal antibody or an antigen-binding fragment thereof.

8. The pharmaceutical composition according to any one of claims 1-7, wherein the antibody-drug conjugate has a structure as shown in the conjugate A below: 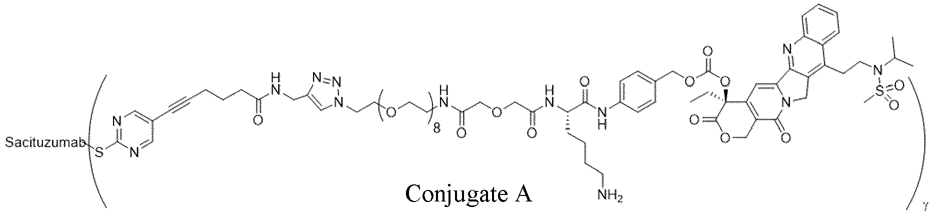 wherein γ is selected from 1-10.

9. The pharmaceutical composition according to claim 7 or 8, wherein the γ is selected from 3-8 (e.g. 3, 4, 5, 6, 7 or 8).

10. The pharmaceutical composition according to any one of claims 1-9, comprising the following components:
the antibody-drug conjugate, histidine hydrochloride, histidine, sucrose, and polysorbate 20.

11. The pharmaceutical composition according to claim 10, wherein the antibody-drug conjugate is a conjugate A.

12. The pharmaceutical composition according to any one of claims 1-11, having a pH in a range of 4.5-7.0.

13. The pharmaceutical composition according to any one of claims 1-11, having a pH in a range of 5.0-6.5.

14. The pharmaceutical composition according to any one of claims 1-11, having a pH in a range of 5.4-6.3.

15. The pharmaceutical composition according to any one of claims 1-11, having a pH in a range of 5.7-6.3.

16. The pharmaceutical composition according to any one of claims 1-11, having a pH in a range of 6.0 ± 0.3.

17. The pharmaceutical composition according to any one of claims 1-16, comprising 5-40 mg/ml of the antibody-drug conjugate, 0.1-3.5 mg/ml of the histidine, 0.1-5.5 mg/ml of the histidine hydrochloride, 30-150 mg/ml of the lyoprotectant, and/or 0.01-0.50 mg/ml of the surfactant.

18. The pharmaceutical composition according to any one of claims 1-16, **characterized by** one or more of the following:
(1) a concentration of the antibody-drug conjugate is 15-25 mg/ml;
(2) a concentration of the histidine is 0.4-1.2 mg/ml;
(3) a concentration of the histidine hydrochloride is 0.5-2.0 mg/ml;
(4) a concentration of the lyoprotectant is 60-120 mg/ml; and
(5) a concentration of the surfactant is 0.05-0.30 mg/ml.

19. The pharmaceutical composition according to any one of claims 1-17, **characterized by** one or more of the following:
(1) a concentration of the antibody-drug conjugate is 18-22mg/ml;
(2) a concentration of the histidine is 0.6-0.8 mg/ml;
(3) a concentration of the histidine hydrochloride is 1.0-1.3 mg/ml;
(4) a concentration of the lyoprotectant is 70-90 mg/ml; and
(5) a concentration of the surfactant is 0.15-0.25 mg/ml.

20. The pharmaceutical composition according to any one of claims 1-19, wherein the pharmaceutical composition contains 20 mg/ml of a conjugate A, 1.1 mg/ml of the histidine hydrochloride, 0.73 mg/ml of the histidine, 80 mg/ml of the sucrose, and 0.2 mg/ml of the polysorbate 20.

21. The pharmaceutical composition according to any one of claims 1-20, which is in unit dosage form.

22. The pharmaceutical composition according to claim 21, wherein the unit dosage form contains 40-400 mg, e.g. 200 mg of the antibody-drug conjugate.

23. The pharmaceutical composition according to any one of claims 1-22, which is a lyophilized preparation, e.g. lyophilized powder.

24. A solid preparation obtained by lyophilizing the pharmaceutical composition according to any one of claims 1-22.

25. The solid preparation according to claim 24, wherein the lyophilizing comprises the following steps:
adding the antibody-drug conjugate, the buffer system, the lyoprotectant and the surfactant into water for injection for lyophilizing to obtain the solid preparation.

26. The solid preparation according to claim 24 or 25, wherein the lyophilizing comprises feeding, pre-freezing, vacuuming, subliming and desorption drying steps.

27. The solid preparation according to claim 26, wherein the pre-freezing is conducted at - 50°C-0°C for a time period of 400-800 minutes.

28. The solid preparation according to claim 26 or 27, wherein the subliming is conducted at -30-0°C and a pressure of 0-100 pa for a time period of 2,000-5,000 minutes.

29. The solid preparation according to any one of claims 26-28, wherein the desorption drying is conducted at 0 - -50°C and a pressure of 0-100 pa for a time period of 200-2,000 minutes.

30. A method for preparing the pharmaceutical composition according to any one of claims 1-23, comprising the following steps:
adding an antibody-drug conjugate, a buffer system, a lyoprotectant and a surfactant into water for injection for lyophilizing to obtain the pharmaceutical composition.

31. The preparation method according to claim 30, wherein the lyophilizing comprises feeding, pre-freezing, vacuuming, subliming and desorption drying steps.

32. The preparation method according to claim 31, wherein the pre-freezing is conducted at - 50°C-0°C for a time period of 400-800 minutes.

33. The preparation method according to claim 31 or 32, wherein the subliming is conducted at -30-0°C and a pressure of 0-100 pa for a time period of 2,000-5,000 minutes.

34. The preparation method according to any one of claims 31-33, wherein the desorption drying is conducted at 0 - -50°C and a pressure of 0-100 pa for a time period of 200-2,000 minutes.

35. A reconstituted liquid preparation obtained by redissolving the pharmaceutical composition according to any one of claims 1-23 or the solid preparation according to any one of claims 24-29 in a solvent.

36. The reconstituted liquid preparation according to claim 35, wherein the solvent is selected from water for injection, normal saline and 5% dextrose in water.

37. The reconstituted liquid preparation according to claim 35, wherein the solvent is water for injection.

38. Use of the pharmaceutical composition according to any one of claims 1-23, the solid preparation according to any one of claims 24-29, or the reconstituted liquid preparation according to any one of claims 35-37 in preparation of a drug for treating tumors.

39. The use according to claim 38, wherein the tumor disease is an unresectable locally advanced or metastatic solid tumor for which standard treatment fails, or there is no standard treatment scheme, or standard treatment is not applicable at this stage.

40. The use according to claim 38 or 39, wherein the tumor is selected from breast cancer, gastric cancer, lung cancer, ovarian cancer, urothelial cancer, esophageal cancer, liver cancer, colorectal cancer, cervical cancer, endometrial cancer, pancreatic cancer, bladder cancer, or brain tumor; and preferably breast cancer (e.g. triple-negative breast cancer or Her2-positive breast cancer), ovarian cancer (e.g. ovarian epithelial cancer), gastric cancer, lung cancer, pancreatic cancer, bladder cancer, or urothelial cancer; and more preferably, the tumor disease is triple-negative breast cancer, Her2-positive breast cancer, ovarian cancer, gastric cancer, lung cancer or pancreatic cancer.

41. The use according to any one of claims 38-40, wherein the tumor is triple-negative breast cancer, Her2-positive breast cancer, ovarian cancer or gastric cancer.

42. A method for treating tumors, comprising the step of administering to a subject a therapeutically effective amount of the pharmaceutical composition according to any one of claims 1-23, the solid preparation according to any one of claims 24-29, or the reconstituted liquid preparation according to any one of claims 35-37.

43. The method according to claim 42, wherein the tumor disease is an unresectable locally advanced or metastatic solid tumor for which standard treatment fails, or there is no standard treatment scheme, or standard treatment is not applicable at this stage.

44. The method according to claim 42 or 43, wherein the tumor is selected from breast cancer, gastric cancer, lung cancer, ovarian cancer, urothelial cancer, esophageal cancer, liver cancer, colorectal cancer, cervical cancer, endometrial cancer, pancreatic cancer, bladder cancer, or brain tumor; and preferably breast cancer (e.g. triple-negative breast cancer or Her2-positive breast cancer), ovarian cancer (e.g. ovarian epithelial cancer), gastric cancer, lung cancer, pancreatic cancer, bladder cancer, or urothelial cancer; and more preferably, the tumor disease is triple-negative breast cancer, Her2-positive breast cancer, ovarian cancer, gastric cancer, lung cancer or pancreatic cancer.

45. The method according to any one of claims 42-44, wherein the tumor is triple-negative breast cancer, Her2-positive breast cancer, ovarian cancer or gastric cancer.
